**Europäisches Patentamt**

**European Patent Office**

**Office européen des brevets**

(19)

(11) Veröffentlichungsnummer: **0 171 792**
**B1**

(12)

# EUROPÄISCHE PATENTSCHRIFT

(45) Veröffentlichungstag der Patentschrift:
**22.06.88**

(51) Int. Cl.⁴: **C 07 C 37/16**

(21) Anmeldenummer: **85110136.0**

(22) Anmeldetag: **13.08.85**

(54) Verfahren zur ortho-Methylierung von Phenolen und Katalysator zur ortho-Methylierung.

(30) Priorität: **17.08.84 DE 3430222**
**22.06.85 DE 3522345**

(43) Veröffentlichungstag der Anmeldung:
**19.02.86 Patentblatt 86/8**

(45) Bekanntmachung des Hinweises auf die Patenterteilung:
**22.06.88 Patentblatt 88/25**

(84) Benannte Vertragsstaaten:
**AT BE DE FR GB IT NL SE**

(56) Entgegenhaltungen:
**EP - A - 0 154 830**

**CHEMICAL ABSTRACTS, Band 94, Nr. 13, 30. März 1981,
p. 707, ref. no. 103010y, Columbus, Ohio, US
PATENTS ABSTRACTS OF JAPAN, vol. 4, no. 190,
December 26, 1980, p. 672 (C-37)**

(73) Patentinhaber: **HÜLS AKTIENGESELLSCHAFT, - RSP
Patente / PB 15 - Postfach 13 20, D-4370 Marl 1 (DE)**

(72) Erfinder: **Voges, Heinz-Werner, Dr., Im Gorden 45,
D-4270 Dorsten 21 (DE)**
Erfinder: **Schmidt, Arno Siegmar, Dr.,
Kristian-Sand-Strasse 15, D-4400 Münster (DE)**

**Beschreibung**

Die Erfindung betrifft ein Verfahren zur selektiven ortho-Methylierung von Phenolen, die in mindestens einer ortho-Stellung ein Wasserstoffatom besitzen, durch Umsetzung dieser Phenole mit Methanol in der Gasphase bei 270 bis 400 °C in Gegenwart eines Katalysators, der zum überwiegenden Teil aus dem Oxid des dreiwertigen Eisens ($Fe_2O_3$) oder Mischoxiden des drei- und zweiwertigen Eisens besteht und erfindungsgemäss ausserdem eine Kombination der Oxide des Wolframs mit Oxiden der Erdalkalimetalle Magnesium, Calcium, Barium, mit Oxiden der Seltenerdmetalle Lanthan und Cer oder mit dem Oxid des Mangans enthält.

Diese ternären Oxidgemisch-Katalysatoren weisen eine hohe Aktivität und Selektivität zur ortho-Alkylierung über längere Zeiträume auf.

Nach dem erfindungsgemässen Verfahren können beispielsweise mit hohen Ausbeuten ortho-Kresol aus Phenol, 2.6-Dimethylphenol aus Phenol und/oder ortho-Kresol und 2.3.6-Trimethylphenol aus meta-Kresol hergestellt werden.

Die Verwendung von reinem Eisenoxid $Fe_2O_3$ als Katalysator der ortho-Methylierung von Phenol ist in der GB-PS 717 588 beschrieben. Es hat bei bereits verhältnismässig niedriger Temperatur eine hohe Alkylierungsaktivität, jedoch sind sowohl die Selektivität zur ortho-Alkylierung als auch die Standzeit für die technische Verwendung unzureichend.

Die noch mangelhafte Eignung des reinen Eisenoxids hat man durch weitere oxidische Komponenten zu beheben versucht. Zahlreiche Katalysator-Kompositionen sind bekannt geworden, die neben dem Hauptbestandteil Eisenoxid Chromoxid, $Cr_2O_3$, enthalten.

In der DE-OS 2 428 056 (= GB-PS 1 428 057) werden Katalysatoren der Zusammensetzung $Fe_2O_3/Cr_2O_3/(SiO_2)/K_2O$ mit dem beispielhaften Metallatomverhältnis $100 : 1 : (10^{-4}) : 2 \cdot 10^{-3}$ beschrieben. Ein binärer Eisenoxid-/Chromoxid-Katalysator ist aus der JP-Patentveröffentlichung 76012-610 bekannt. Ein neben Eisen- und Chromoxid noch Indium-, Silicium- und Kaliumoxid enthaltender Katalysator wird in der JP-PS 58 109 436 beansprucht. Ferner kennt man überwiegend aus Eisenoxid bestehende Katalysatoren der Zusammensetzung Fe/Cr/Ge (oder Ga, Nb) (Europäische Patentveröffentlichung 0 019 476), Fe/Cr/Ge(oder Si)/Mn (oder La) (DE-PS 3 103 839 (= US-PS 4 359 591 und GB-PS 2 072 674), Fe/Cr/Sn/K (US-PS 4 227 024), Fe/Cr/Zr (oder Cd und andere)/$K_2CO_3$ (Europäische Patentveröffentlichung 0 050 937), Fe/Cr/Ce (Europäische Patentveröffentlichung 0 081 647).

Der Erfindung liegt die Aufgabe zugrunde, ein katalytisches Verfahren zur ortho-Methylierung von Phenolverbindungen, die mindestens ein Wasserstoffatom in ortho-Stellung besitzen, in der Gasphase zu entwickeln, das in einfacher Weise bei hoher Aktivität, Selektivität und Standzeit des Katalysators durchgeführt werden kann.

Diese Aufgabe wird erfindungsgemäss entsprechend den Angaben der Patentansprüche gelöst.

Die bisher aufgeführten Eisenoxid-Kombinationen des Standes der Technik enthalten als eine essentielle Komponente das Oxid des dreiwertigen Chroms.

In der JP-A-80 129235 (Chem. Abstr. 94, 103010 y (1981) wird unter anderem auch ein binärer Eisenoxid-Wolframoxid-Kontakt beschrieben, der zwar bereits eine hohe Selektivität von 97,5% hinsichtlich der Orthomethylierung besitzt (s. Tab. 2 der japan. Patentveröffentlichung), der aber in bezug auf den erreichten Phenolumsatzgrad und die Selektivität hinsichtlich der Bildung von 2.6-Xylenol (= 2.6-Dimethylphenol) schlecht abschneidet. Die relative Schwäche hinsichtlich der Bildung von 2.6-Dimethylphenol wird auch offensichtlich, wenn man unter sonst gleichen Bedingungen binäre Fe-/Cr-, Fe-/Mo- und Fe/W-Oxidkontakte direkt miteinander vergleicht (siehe Vergleichsbeispiele A, B, C dieser Erfindung).

Überraschend wurde nun gefunden, dass man auf der Basis des Fe-/W-Oxid-Systems zu technisch brauchbaren Katalysatoren mit der erforderlichen hohen Selektivität hinsichtlich der 2.6-Dimethylphenol-Bildung gelangt, wenn man Eisenoxid, z.B. $Fe_2O_3$, und Wolframoxid $WO_3$ in Verbindung mit den Oxiden der Erdalkalimetalle Magnesium, Calcium oder Barium, der Seltenerdmetalle Lanthan oder Cer oder des Mangans einsetzt.

Auch das Fe-/Mo-oxid-System kann in Verbindung mit den genannten Drittkomponenten (c) verbessert werden; die Verbesserung im Vergleich zum binären Fe-/Mo-oxid-Kontakt ist aber geringer als beim Fe-/W-oxid-Kontakt.

Die erfindungsgemässen ternären Oxidgemische haben Metallatomverhältnisse Fe/W/Erdalkalimetall bzw. Seltenerdmetall bzw. Mangan wie 100/0.2 bis 10/0.2 bis 10, bevorzugt von 100/0.4 bis 4/0.4 bis 4. Das Oxidgemisch aus Eisenoxid, Wolframoxid und Magnesiumoxid setzt man bevorzugt im Metallatomverhältnis 100/0.2 bis 2/0.2 bis 2 ein.

Die Herstellung der erfindungsgemässen ternären Katalysatoren erfolgt nach bekannten Verfahren, z.B. durch innige Vermischung der oxidischen Komponenten oder bevorzugt durch Copräcipitation der Hydroxide bzw. Oxide aus wässrigen Lösungen der Metallsalze, wobei Wolfram z.B. als Ammoniumwolframat eingesetzt werden kann, mit Basen wie wässriger Ammoniaklösung, Alkalilaugen oder Alkali(bi)carbonatlösungen. Der Eisen-hydroxid, beispielsweise -(III)-hydroxid-Niederschlag, der die Oxide/Hydroxide der Zusatzkomponenten in gleichmässiger Verteilung enthält, wird mit Wasser von Femdionen und evtl. überschüssiger Base freigewaschen, bei 130 °C getrocknet und zu Pulver vermahlen. Das pulverförmige Oxidgemisch kann, gegebenenfalls nach Zugabe von bis zu 10 Gew.-% eines inerten Zuschlagstoffes, wie z.B. Graphit, zu Formkörpern geeigneter Grösse – Tabletten oder Strangpresslinge – verpresst werden. Nach Calcinierung bei Temperaturen von 300 bis 500 °C sind die Katalysator-Formkörper für die in der Gasphase stattfindende Umsetzung der Phenole mit Methanol vorbereitet. Die Formkörper werden in von aussen thermostatierbare Reaktionsrohre mit Innendurchmessern von bis zu 50 mm, bevorzugt bis zu 32 mm, eingefüllt. Die Reaktionspartner (phenolische Verbindung und Methanol) werden im bestimmten Molverhältnis vor Eintritt in das mit Katalysator gefüllte Reaktionsrohr verdampft, zweckmässig in der Art, dass eine Lösung der phenolischen Verbindung in der berechneten Menge Methanol einem Verdampfer/Vorheizer zugeführt wird und das aus diesem austretende Dampfgemisch über den Katalysator geleitet wird.

Die Reaktion findet unter folgenden Bedingungen statt:

Molverhältnis phenolische Verbindung zu Methanol wie 1 : 1 bis 1 : 10, Raumgeschwindigkeit (LHSV), berechnet für das Reaktanden-Gemisch vor der Verdampfung, 0.05 bis 3 h$^{-1}$

Reaktionstemperatur 270 bis 400 °C, bevorzugt 300 bis 350 °C

Druck 1 bis 4 bar absolut.

Unter den genannten Reaktionsbedingungen gelingt es, einen praktisch vollständigen Umsatz der eingesetzten phenolischen Verbindung herbeizuführen, wobei sich die gewünschten ortho-Alkylierungsprodukte in hoher Selektivität bilden.

Wie die nachstehend aufgeführten Beispiele belegen, stellen die ternären Wolframoxid enthaltenden Oxidgemische Katalysatoren dar, mit denen bei praktisch vollständigem Phenolumsatzgrad und unter Erhaltung einer hohen Gesamtselektivität die Selektivität zum 2.6-Dimethylphenol im Vergleich zum binären Fe-/W-oxid von ca. 75% überraschenderweise auf 85 bis 90% erhöht wird. Die erfindungsgemässen ternären Kontakte zeigen eine lange Lebensdauer, die über die gesamte Versuchszeit von mehr als 2 000 Stunden erhalten bleibt.

Vergleichsbeispiele A, B und C

Binäre Katalysatoren Fe/Cr, Fe/Mo und Fe/W werden durch Copräcipitation der Hydroxide/Oxide mit 10%iger wässriger NH$_3$-Lösung aus wässrigen Lösungen von je 404 g Fe (NO$_3$)$_3$ · 9H$_2$O mit Vgl.-Beispiel A: 2 g Cr(NO$_3$) · 9H$_2$O
Vgl.-Beispiel B: 0,87 g (NH$_4$)$_6$Mo$_7$O$_{24}$ · 4H$_2$O
Vgl.-Beispiel C: 1.3 g (NH$_4$)$_{10}$W$_{12}$O$_{41}$ · 7H$_2$O
hergestellt. Die, wie oben beschrieben, vorbereiteten Katalysatoren werden in Reaktionsrohre (D$_i$ = 22 mm) eingefüllt und in einem Thermostatenbad temperiert. Phenol-/Methanoldämpfe (Molverhältnis 1 : 5) werden bei Normaldruck über die Kontakte geleitet. Die LHSV beträgt in allen drei Fällen 0.09 h$^{-1}$, die Reaktionstemperatur 323 °C. Die die Reaktionsrohre verlassenden Produktdämpfe werden in durch Wasser gekühlten Kühlern kondensiert, die so verflüssigten Produktgemische durch Gaschromatographie analysiert. Mit den binären Katalysatoren erhält man nach einer Laufzeit von 76 Stunden die folgenden Ergebnisse:

| Vgl.-Bei-spiel | Oxid-Komponenten Metallatomverh. | Phenol-Umsatz (%) | Selektivität (%) zu | | ortho-Methy-lierung | Trimethyl-phenolen |
|---|---|---|---|---|---|---|
| | | | o-Kresol | 2.6-DMP [+]) | | |
| A | Fe/Cr 100/0.5 | 99.9 | 5.0 | 92.7 | 97.7 | 2.3 |
| B | Fe/Mo 100/0.5 | 99.9 | 11.9 | 84.9 | 96.8 | 3.2 |
| C | Fe/w 100/0.5 | 99.6 | 21.4 | 75.0 | 96.4 | 3.6 |

[+]) 2.6–DMP = 2.6–Dimethylphenol

Die Vergleichsbeispiele zeigen, dass der binäre Fe/Mo-Kontakt eine schwächere Aktivität zur gewünschten Zweitalkylierung (2.6-Dimethylphenolbildung) hat als der binäre Fe/Cr-Kontakt und dass der binäre Fe/W-Kontakt schwächere Zweitalkylierungsaktivität hat als der Fe/Mo-Kontakt.

Vergleichsbeispiele 1 bis 4 (nicht erfindungsgemäss)

Die folgenden Vergleichsbeispiele stellen ternäre Katalysatoren auf Basis Fe/Mo dar, die zusätzlich eine weitere oxidische Komponente enthalten. Sie werden nach der Copräcipitationsmethode bereitet aus je 404 g Fe(NO$_3$)$_3$ · 9H$_2$O und 0.87 g (NH$_4$)$_6$Mo$_7$O$_{24}$ · 4H$_2$O sowie

Beispiel 1: 1.18 g Ca(NO$_3$)$_2$ · 4H$_2$O

Beispiel 2: 1.24 g Ba(NO$_3$)$_2$ · H$_2$O
Beispiel 3: 1.45 g Ce(NO$_3$)$_3$ · 6H$_2$O
Beispiel 4: 1.44 g Mn(NO$_2$)$_2$ · 6H$_2$O

Die durch Fällung mit Ammoniakwasser aus wässriger Lösung erhaltenen Niederschläge werden nach Filtration, Waschung und Trocknung zu 3 × 3 mm Tabletten verpresst, die anschliessend bei 450 °C im Luftstrom calciniert werden. Phenol-/Methanol-Dampfgemische (Molverhältnis 1 : 5) werden bei 1 bar über die Katalysatoren geleitet, die sich in Reaktionsrohren mit einem Innendurchmesser von 22 mm befinden. Die Raumgeschwindigkeit des Phenol-/Methanol-Einsatzgemisches vor der Verdampfung beträgt einheitlich 0.08 h$^{-1}$. Nach einer Versuchszeit von 100 h erhält man die folgenden Ergebnisse:

| Vergleichs-Bspl. | Katalysator Atomverhältnis | Reaktions-temperatur (°C) | Phenol-Umsatz (%) | Selektivität (%) zu | | | |
| | | | | o-Kresol | 2.6–DMP[+]) | ortho-Methylierung | Trimethyl-phenolen |
| --- | --- | --- | --- | --- | --- | --- | --- |
| 1 | Fe/Mo/Ca 100/0.5/0.5 | 324 | 99.7 | 11.7 | 82.4 | 94.1 | 4.2 |
| 2 | Fe/Mo/Ba 100/0.5/0.5 | 324 | 99.4 | 16.4 | 80.4 | 96.8 | 2.1 |
| 3 | Fe/Mo/Ce 100/0.5/0.33 | 320 | 99.3 | 12.2 | 85.4 | 97.6 | 2.0 |
| 4 | Fe/Mo/Mn 100/0.5/0.5 | 324 | 99.4 | 15.5 | 82.7 | 98.2 | 1.3 |

[+]) 2.6–DMP = 2.6–Dimethylphenol
para-Kresol bildet sich mit Selektivitäten von <0.1%

Vergleichsbeispiel 5 (nicht erfindungsgemäss)

Der Katalysator des Vergleichsbeispiels 3 (Fe/Mo/Ce) wird einem Langzeittest über 1 000 h unterworfen. Dabei wird die Reaktionstemperatur bei 1 bar schrittweise in dem Masse erhöht, dass der Phenolumsatz grösser als 99% bleibt.

| Laufzeit (h) | 70 | 380 | 730 | 1150 |
| --- | --- | --- | --- | --- |
| Reaktionstemperatur (°C) | 319 | 322 | 324 | 326 |
| Phenolumsatz (%) | 99.6 | 99.7 | 99.5 | 99.6 |
| Selektivität zur ortho-Methylierung (%) | 97.4 | 97.9 | 98.2 | 98.5 |
| davon zu ortho-Kresol (%) | 10.0 | 10.2 | 10.3 | 10.5 |
| 2.6–DMP (%) | 87.4 | 87.7 | 87.9 | 88.0 |
| Trimethylphenolen | 2.2 | 2.0 | 1.2 | 1.1 |

Über die gesamte Laufzeit wird para-Kresol nur in Selektivitäten von <0.1% gebildet. Der Katalysator ist nach der Versuchszeit von über 1 000 h noch voll aktiv bei hoher Selektivität zu ortho-Methylierungsprodukten.

Beispiele 6 bis 9

Erfindungsgemässe ternäre Katalysatoren auf Basis Fe/W werden nach der Copräcipitationsmethode hergestellt aus je 404 g $Fe(NO)_3 \cdot 9H_2O$, 1.32 g $(NH_4)_{10}W_{12}O_{41} \cdot 7H_2O$ sowie

Beispiel 6: 1.28 g $Mg(NO_3)_2 \cdot 6H_2O$
Beispiel 7: 2.17 g $La(NO_3)_3 \cdot 6H_2O$
Beispiel 8: 1.30 g $Ba(NO_3)_2 \cdot H_2O$
Beispiel 9: 2.17 g $Ce(NO_3)_3 \cdot 6H_2O$.

Über die bei 450 °C calcinierten Katalysatoren (Tabletten 3 × 3 mm) werden unter gleichen Bedingungen wie in den Beispielen 1 bis 4 Phenol/Methanoldämpfe (Molverhältnis 1 : 5) geleitet. Nach einer Versuchszeit von 100 h erhält man die folgenden Ergebnisse:

| Bspl. | Katalysator Atomverhältnis | Reaktions-temperatur (°C) | Phenol-Umsatz (%) | Selektivität (%) zu | | | Methanol-Verlust [+]) (%) |
| | | | | o-Kresol | 2.6-DMP | Trimethyl-phenolen | |
| --- | --- | --- | --- | --- | --- | --- | --- |
| 6 | Fe/W/Mg 100/0.5/0.5 | 326 | 99.7 | 7.3 | 85.1 | 6.6 | 34 |
| 7 | Fe/W/La 100/0.5/0.5 | 320 | 99.6 | 8.0 | 89.3 | 1.9 | 39 |
| 8 | Fe/W/Ba 100/0.5/0.5 | 318 | 99.9 | 5.4 | 93.1 | 1.1 | 32 |
| 9 | Fe/W/Ce 100/0.5/0.5 | 320 | 99.1 | 7.2 | 90.0 | 2.0 | 40 |

[+]) Methanol-Verlust in Form von gasförmigen Zersetzungsprodukten ($H_2$, CO, $CO_2$) in % vom Einsatz.
para-Kresol ist in den Reaktionsprodukten nur in Spuren unter 0.1 Gew.-% enthalten.

Beispiel 10

Ein Katalysator, bestehend aus Eisen-, Wolfram- und Bariumoxid, in den Metallatomverhältnissen 100 : 2 : 0.6, der nach der Copräcipitationsmethode hergestellt worden ist, wird unter Bedingungen wie im Vergleichsbeispiel 5 einem Test über 2 000 h unterworfen.

Dabei erhält man die folgenden Ergebnisse:

| Laufzeit (h) | 20 | 170 | 242 | 506 | 1203 | 2076 |
|---|---|---|---|---|---|---|
| Reaktionstemperatur (°C) | 314 | 319 | 322 | 324 | 324 | 328 |
| Phenolumsatz (%) | 99.6 | 99.8 | 99.8 | 99.7 | 99.0 | 99.7 |
| Selektivität zur ortho-alkylierung (%) | 97.8 | 98.3 | 98.4 | 98.4 | 98.7 | 98.2 |
| davon zu o-Kresol (%) | 11.7 | 6.7 | 7.4 | 12.7 | 9.9 | 11.1 |
| 2.6-DMP (%) | 86.1 | 91.6 | 91.0 | 85.7 | 88.8 | 87.1 |
| Trimethylphenolen (%) | 1.3 | 1.0 | 0.95 | 0.87 | 0.90 | 1.1 |
| Methanol-Verlust (%) | 28 | 32 | 33 | 34 | 34 | 35 |

Die Raumgeschwindigkeit (LHSV) beträgt während der gesamten Laufzeit 0.08 $h^{-1}$. Am Ende der Versuchszeit ist der Katalysator noch voll aktiv und zeigt eine hohe Selektivität. para-Kresol bildet sich nur in Spuren von unter 0.1 Gew.-%. Die Methanol-Verluste sind verhältnismässig gering.

Beispiel 11

Nach der Copräcipitationsmethode wird ein Katalysator aus 404 g $Fe(NO_3)_3 \cdot 9H_2O$, 2,64 g $(NH_4)_{10}W_{12}O_{41} \cdot 7H_2O$, 1,30 g $Ba(NO_3)_2 \cdot H_2O$ und 1,44 g Mn $(NO_3)_2 \cdot H_2O$ bereitet. Zur Fällung des Hydroxid-/Oxid-Niederschlages aus der wässrigen Lösung der Salze wird wässrige Ammoniak-Lösung benutzt. Das Metallatomverhältnis Fe/W/Ba/Mn beträgt 100/1/0,5/0,5. Das nach Filtration mit Wasser gewaschene, dann bei 130 °C getrocknete und zu Tabletten (3 × 3 mm) verpresste Gemisch wird bei 430 °C calciniert. Phenol-/Methanoldämpfe (Molverhältnis 1 : 5) werden mit LHSV = 0,1 $h^{-1}$ bei 1 bar über den Katalysator geleitet, der sich in einem Reaktionsrohr mit einem Innendurchmesser von 22 mm befindet. Nach einer Laufzeit von 100 h beträgt der Phenol-Umsatz laut gaschromatografischer Analyse des Reaktionsproduktes 99,4%, wobei die Reaktorinnentemperatur zu diesem Zeitpunkt bei 325,5 °C gehalten wird. Die Selektivitäten zu ortho-Kresol, 2,6-Dimethylphenol bzw. Trimethylphenolen betragen 8,2%, 90,1% bzw. 1,3%.

Vergleichsbeispiel 12 (nicht erfindungsgemäss)

Aus einer wässrigen Lösung von 404 g Fe-$(NO_3)_3 \cdot 9H_2O$, 2,61 g $(NH_4)_6Mo_7O_{24} \cdot 4H_2O$, 1,45 g $Ce(NO_3)_3 \cdot 6H_2O$ und 5,76 g $Mn(NO_3)_2 \cdot 6H_2O$ (Metallatomverhältnis Fe/Mo/Ce/Mn = 100/1,5/0,5/2,0) werden mit 10%iger wässriger Natronlauge die Metallhydroxide/-oxide ausgefällt. Der Niederschlag wird abfiltriert und mit Wasser gewaschen, danach getrocknet, pulverisiert, zu 3 × 3 mm-Tabletten verpresst und diese bei 420 °C calciniert. Phenol-/Methanoldämpfe (Molverhältnis 1 : 5) werden mit einer Raumgeschwindigkeit LHSV = 0,09 $h^{-1}$ über den Katalysator, der sich in einem Reaktionsrohr mit dem inneren Durchmesser von 22 mm befindet, geleitet (Druck 1 bar).

Nach einer Laufzeit von 130 h und bei einer Reaktorinnentemperatur von 322 °C beträgt der Phenolumsatz 99,4% und die Selektivität zu ortho-Kresol 13,1%, zu 2,6-Dimethylphenol 84,2% und zu Trimethylphenolen 1,8%. Im Reaktionsprodukt befindet sich para-Kresol nur in Spuren von <0,1 Gew.-%.

Beispiele 13 bis 15

Erfindungsgemässe ternäre Katalysatoren auf Basis Fe/W/Mg werden nach der Copräzipitationsmethode hergestellt aus je 250 g $Fe(NO_3)_3 \cdot 9 H_2O$, 0,80 g $Mg(NO_3)_2 \cdot 6H_2O$ sowie
Beispiel 13: 0,81 g 5 $(NH_4)_2O \cdot 12 WO_3 \cdot 7H_2O$
Beispiel 14: 0,66 g 5 $(NH_4)_2O \cdot 12 WO_3 \cdot 7H_2O$
Beispiel 15: 0,99 g 5 $(NH_4)_2O \cdot 12 WO_3 \cdot 7H_2O$

Die Fällung und Trocknung erfolgt wie bei den Beispielen 1 bis 4, die Calcinierung wird bei 400 °C durchgeführt.

Die Beispiele 13 bis 15 werden bei einem Eduktkomponentenverhältnis von Phenol/Methanol = 1/5 entsprechend den Angaben der Tabelle durchgeführt.

Beispiele 16 und 17

Die Darstellung der Katalysatoren erfolgt wie unter Beispiel 13 bis 15 aufgeführt. Die Durchführung erfolgt mit gleichem Phenol/Methanol-Verhältnis wie oben und weiter entsprechend den Angaben in der Tabelle.

| Beispiel | Atomverhältnis Fe/W/Mg | Katalysatorlaufzeit (h) | LHSV | Reaktortemp. (°C) | Druck (bar) | Phenol-Umsatz (%) |
|---|---|---|---|---|---|---|
| 13 | 100/0.5/0.5 | 150 | 0,1 | 323 | 1 | 99,8 |
| 14 | 100/0.4/0.5 | 100 | 0,1 | 323 | 1 | 99,9 |
| 15 | 100/0.6/0.5 | 70 | 0,09 | 323 | 1 | 99,9 |
| 16 | 100/0.5/0.5 | 100 | 0,09 | 323 | 2 | 99,8 |
| 17 | 100/0.5/0.5 | 80 | 0,09 | 323 | 4 | 99,8 |

| Beispiel | % Selektivität zu | | Rest | % CH₃OH Verl. |
|---|---|---|---|---|
| | o-Kresol | 2,6-DMP | | |
| 13 | 6,9 | 87,7 | 4,3 | 35 |
| 14 | 9,7 | 83,0 | 6,3 | 28 |
| 15 | 8,8 | 81,4 | 4,5 | 32 |
| 16 | 7,1 | 84,3 | 5,1 | 28 |
| 17 | 7,3 | 83,9 | 5,5 | 27,5 |

## Patentansprüche

1. Verfahren zur ortho-Methylierung von Phenolen, die mindestens eine freie ortho-Stellung besitzen, durch Umsetzung dieser Phenole mit Methanol im Molverhältnis 1 : 1 bis 1 : 10 bei 270 bis 400 °C in der Gasphase, bei einem Druck von 1 bis 4 bar absolut, Raumgeschwindigkeiten (LHSV) von 0.05 bis 3 h⁻¹ und in Gegenwart von Eisenoxid-Katalysatoren, dadurch gekennzeichnet, dass man die Umsetzung in Gegenwart eines Katalysators durchführt, der aus einer Kombination aus

a) Eisenoxid, b) Wolframoxid und c) einem oder mehreren Oxiden der Elemente Magnesium, Calcium, Barium, Lanthan, Cer, Mangan im Metallatomverhältnis a) zu b) zu c) wie 100 zu 0.2 bis 10 zu 0.2 bis 10 besteht.

2. Verfahren nach Anspruch 1, dadurch gekennzeichnet, dass man einen Katalysator mit Metallatomverhältnis a) zu b) zu c) von 100 zu 0,4 bis 4 zu 0,4 bis 4 einsetzt.

3. Verfahren nach den Ansprüchen 1 und 2, dadurch gekennzeichnet, dass man einen Katalysator einsetzt, der aus einer Kombination aus a) Eisenoxid, b) Wolframoxid und c) Bariumoxid besteht.

4. Verfahren nach den Ansprüchen 1 und 2, dadurch gekennzeichnet, dass man einen Katalysator einsetzt, der aus einer Kombination aus a) Eisenoxid, b) Wolframoxid und C) Magnesiumoxid besteht.

5. Katalysator zur ortho-Methylierung, gekennzeichnet durch die Kombination aus a) Eisenoxid, b) Wolframoxid und c) einem oder mehreren Oxiden der Elemente Magnesium, Calcium, Barium, Lanthan, Cer und Mangan im Metallatomverhältnis a) zu b) zu c) von 100 zu 0.2 bis 10 zu 0.2 bis 10.

6. Katalysator zur ortho-Methylierung nach Anspruch 5, gekennzeichnet durch die Kombination aus a) Eisenoxid, b) Wolframoxid und c) Bariumoxid im Metallatomverhältnis a) zu b) zu c) von 100 zu 0,4 bis 4 zu 0,4 bis 4.

7. Katalysator zur ortho-Methylierung nach Anspruch 5, gekennzeichnet durch die Kombination aus a) Eisenoxid, b) Wolframoxid, und c) Magnesiumoxid im Metallatomverhältnis a) zu b) zu c) von 100 zu 0,2 bis 2 zu 0,2 bis 2.

## Claims

1. A process for ortho-methylation of a phenol which has at least one free ortho-position, by reacting this phenol with methanol in the molar ratio 1 : 1 to 1 : 10 at 270 to 400 °C in the gas phase, at a pressure of 1 to 4 bar absolute, at a space velocity (LHSV) of 0.05 to 3 h⁻¹ and in the presence of an iron oxide catalyst, characterized in that the reaction is carried out in the presence of a catalyst which is composed of a combination of

a) iron oxide, b) tungsten oxide and c) one or more oxides of the elements magnesium, calcium, barium, lanthanum, cerium and manganese in the metal atom ratio a) : b) : c) of 100 : 0.2 to 10 : 0.2 to 10.

2. A process according to claim 1, characterized in that a catalyst is employed in the metal atom ratio a) : b) : c) of 100 : 0.4 to 4 : 0.4 to 4.

3. A process according to any of claims 1 and 2, characterized in that a catalyst is employed which is composed of a combination of a) iron oxide, b) tungsten oxide and c) barium oxide.

4. A process according to any of claims 1 and 2, characterized in that a catalyst is employed which is composed of a combination of a) iron oxide, b) tungsten oxide and c) magnesium oxide.

5. A catalyst for ortho-methylation, characterized by the combination of a) iron oxide, b) tungsten oxide and c) one or more oxides of the elements magnesium, calcium, barium, lanthanum, cerium and manganese in the metal atom ratio a) : b) : c) of 100 : 0.2 to 10 : 0.2 to 10.

6. A catalyst for ortho-methylation according to claim 5, characterized by the combination of a) iron oxide, b) tungsten oxide and c) barium oxide in the metal atom ratio a) : b) : c) of 100 : 0.4 to 4 : 0.4 to 4.

7. A catalyst for ortho-methylation according to claim 5, characterized by the combination of a) iron oxide, b) tungsten oxide and c) magnesium oxide in the metal atom ratio a) : b) : c) of 100 : 0.2 to 2 : 0.2 to 2.

## Revendications

1. Procédé pour l'ortho-méthylation de phénols possédant au moins une position ortho libre, par réaction de ces phénols sur du méthanol dans un rapport molaire de 1 : 1 à 1 : 10, à une température de 270 à 400 °C, en phase gazeuse, sous une pression de 1 à 4 bars absolus, avec des vitesses spatiales (LHSV) de 0,05 à 3 h⁻¹ et en présence de catalyseurs à l'oxyde ferrique, caractérisé par le fait que l'on effectue la réaction en présence d'un catalyseur qui est constitué par une combinaison:

a) d'oxyde ferrique, b) d'oxyde de tungstène et c) d'un ou plusieurs oxydes des éléments que sont le magnésium, le calcium, le baryum, le lanthane, le cérium, le manganèse, dans une proportion d'atomes de métal de a) à b) à c) comme 100 pour 0,2 à 10 pour 0,2 à 10.

2. Procédé selon la revendication 1, caractérisé par le fait que l'on utilise un catalyseur dans une proportion d'atomes de métal a) à b) à c) de 100 pour 0,4 à 4 pour 0,4 à 4.

3. Procédé selon les revendications 1 et 2, caractérisé par le fait que l'on utilise un catalyseur qui est constitué par une combinaison: a) d'oxyde ferrique, b) d'oxyde de tungstène et c) d'oxyde de baryum.

4. Procédé selon les revendications 1 et 2, caractérisé par le fait que l'on utilise un catalyseur qui est constitué par une combinaison: a) d'oxyde ferrique, b) d'oxyde de tungstène et c) d'oxyde de magnésium.

5. Catalyseur d'ortho-méthylation, caractérisé par le fait qu'il est constitué par une combinaison: a) d'oxyde ferrique, b) d'oxyde de tungstène et c) d'un ou plusieurs oxydes des éléments que sont le magnésium, le calcium, le baryum, le lanthane, le cérium, le manganèse dans une proportion d'atomes de métal de a) à b) à c) comme 100 pour 0,2 à 10 pour 0,2 à 10.

6. Catalyseur d'ortho-méthylation selon la revendication 5, caractérisé par le fait qu'il est constitué par une combinaison: a) d'oxyde ferrique, b) d'oxyde de tungstène et c) d'oxyde de baryum comme 100 pour 0,4 à 4 pour 0,4 à 4.

7. Catalyseur d'ortho-méthylation selon la revendication 5, caractérisé par le fait qu'il est constitué par une combinaison: a) d'oxyde ferrique, b) d'oxyde de tungstène et c) d'oxyde de magnésium dans une proportion d'atomes de métal de a) à b) à c) comme 100 pour 0,2 à 2 pour 0,2 à 2.

7